# EUROPEAN PATENT SPECIFICATION

(11) **EP 2 846 740 B1**
(45) Date of publication and mention of the grant of the patent: **01.08.2018**
(21) Application number: 13724471.1
(22) Date of filing: 08.05.2013
(51) Int. Cl.: A61F 2/36, A61F 2/34

(54) **OPTIMAL CONTACT MECHANICS FOR A THA**
OPTIMALE KONTAKTMECHANIK FÜR OBERSCHENKELPROTHESENKOMPONENTEN
MÉCANIQUE DE CONTACT OPTIMAL POUR UN THA

(30) Priority: 08.05.2012 US 201213466944
(43) Date of publication of application: 18.03.2015
(73) Proprietor: DePuy Ireland Unlimited Company, County Cork (IE)
(72) Inventor: KOMISTEK, Richard D., Knoxville, Tennessee 37934 (US)
(74) Representative: Curran, Clair
(86) International application number: PCT/US2013/040107
(87) International publication number: WO 2013/169881

(56) References cited:
- EP-A1- 0 797 964
- EP-A1- 1 574 183
- EP-A2- 0 649 640
- EP-A2- 1 508 315
- WO-A1-00/64384
- WO-A1-2009/118673
- WO-A2-2008/058756
- DE-A1- 2 139 878
- DE-A1- 3 643 815
- FR-A1- 2 785 523
- FR-A1- 2 889 446
- GB-A- 1 573 608
- US-A- 3 510 883
- US-A- 4 032 994
- US-A- 4 911 723
- US-A- 4 960 427
- US-A- 5 047 062
- US-A- 5 593 447
- US-A- 5 702 474
- US-B1- 6 200 350

## Description

### RELATED ART

### FIELD OF THE INVENTION

The present disclosure is directed to components that may be implanted as part of a prosthetic joint such as a hip joint. The closest prior art is document US 5047062 A, which defines the preamble of claim 1.

### BRIEF DISCUSSION OF RELATED ART

It has been stated in numerous publications that the human hip is a ball and socket joint, whereas all three sequential rotations are present, even though the actual shapes of the acetabulum and femoral head of the femur are not pure spheres. The normal hip can experience flexion/extension, internal/external rotation and abduction/adduction rotation, comprising three sequential rotations to perform an activity. Due to the ligamentous constraints, most notably the Iliofemoral ligaments and the femoral head ligament, the femoral head is not allowed to experience significant translation with respect to the acetabulum.

Present day total hip arthroplasty (THA) implants designed so that the femoral neck is either: (1) aligned along a horizontal axis parallel with the ground; or, (2) aligned along an axis that is oriented between thirty to forty-five degrees with respect to a horizontal axis (see FIG. 1, where θ represents the angle of the femoral neck with respect to a horizontal axis or plane). Full systems of femoral stems have been designed with this guiding principle, desiring for the implanted femoral neck to follow the orientation of the anatomical neck (see FIG. 2). Although these implanted stems attempt to maintain the angle of the anatomical neck, one must understand that the anatomical neck is much thicker and patient specific (see FIG. 3).

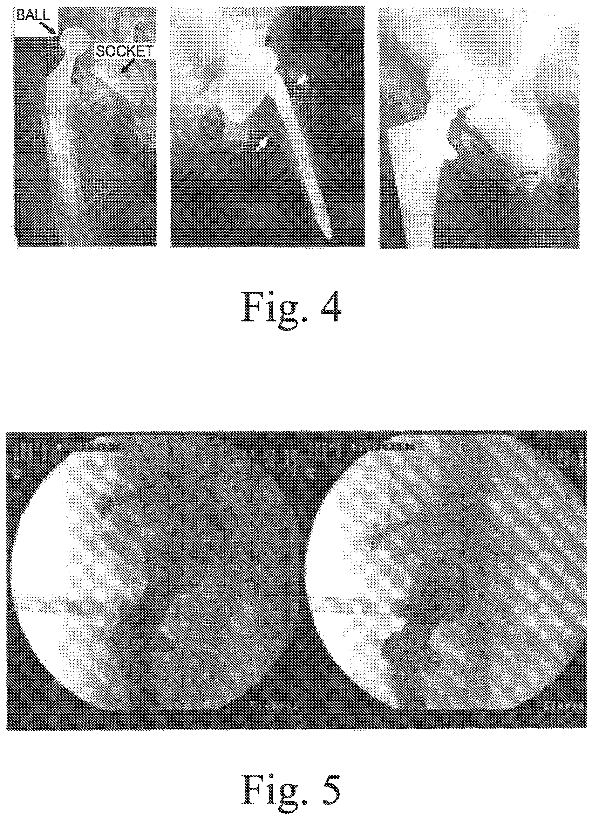

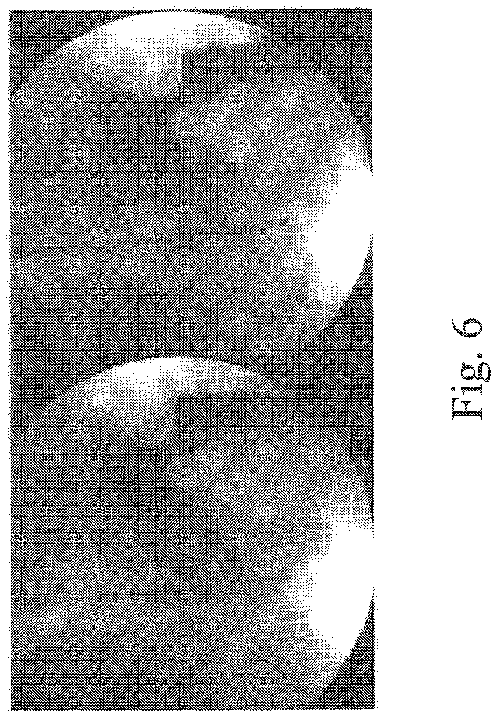

Among the concerns with present day THA implants are: (1) dislocation of the femoral head from the acetabular cup; and, (2) sliding of the femoral head within the acetabular cup. Dislocation is a major concern with present day THA implants and this phenomenon very rarely occurs with anatomical hips. When dislocation occurs with respect to THA implants, the femoral head fully disengages from the acetabular cup (see FIG. 4). Femoral head separation, also very rarely seen in anatomical hips, routinely occurs with THA implants as the femoral head slides on the superolateral aspect of the acetabular cup in the lateral direction (see FIGS. 5 and 6). This phenomenon of femoral head separation occurs during both weight-bearing and non weight-bearing activities and is a major concern with present day THA. This phenomenon occurs in over 95% of all patients having THA. The present inventor hypothesizes that these phenomena (dislocation and femoral head separation) likely occur because the femoral neck axis is not aligned with the weight-bearing axis of the leg (i.e., femur).

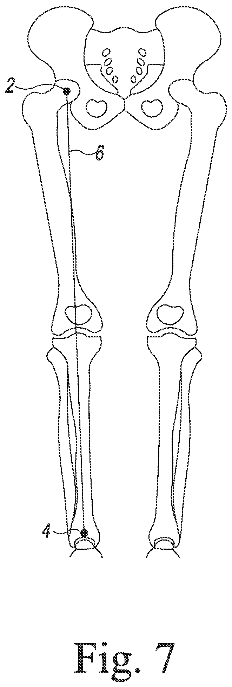

In the natural femur, the weight-bearing axis 6 is presumed to be the mechanical axis (see FIG. 7). The mechanical axis is defined by a first point 2 located at the center of the femoral head sphere and a second point 4 located at the center of the ankle joint. It is therefore, assumed that this axis 6, in a normal, healthy leg, also passes either through the center of the knee or medial of the knee center, possibly even through the medial condyle. The mechanical axis 6 is therefore assumed to bear the weight of the upper body as this axis is aligned with the direction of the weight-bearing force applied at the hip joint.

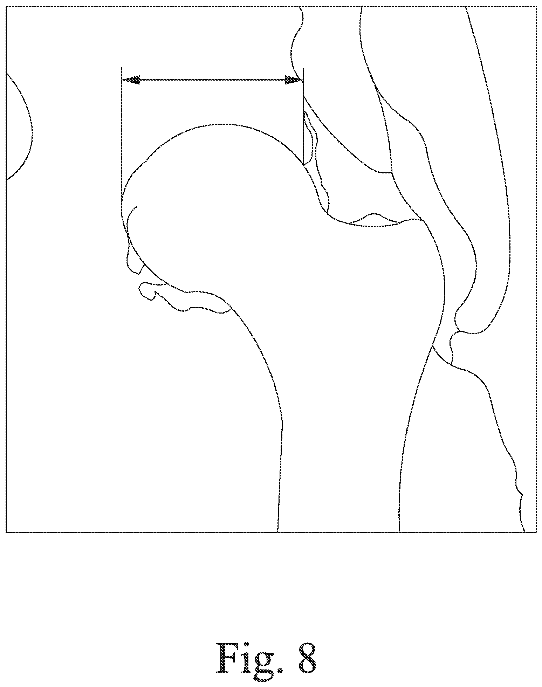

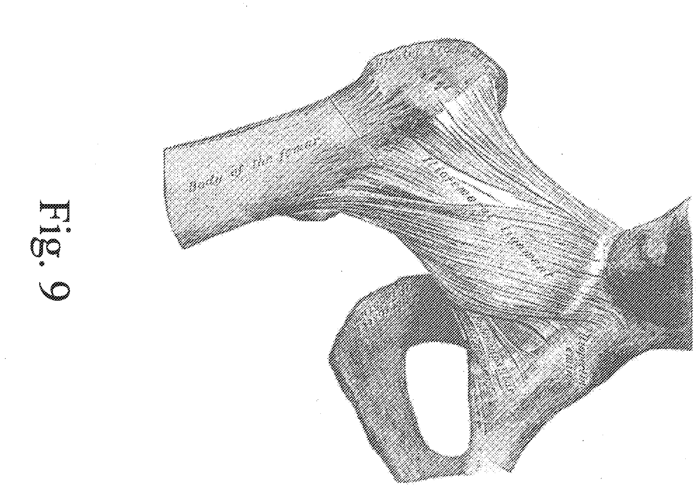

In the normal hip, the large size of the femoral head allows for the hip joint force to be dispersed over a large area, leading to minimized contact stresses on the femoral head (see FIG. 8). Also, the normal hip has a supporting ligament structure that maintains femoral head contact within the acetabulum during all activities (see FIG. 9). Therefore, the large contact area is maintained in the anatomical hip joint, allowing for the bearing surface force to be displaced over this large contact area, leading to contact stresses that are well below destructive levels.

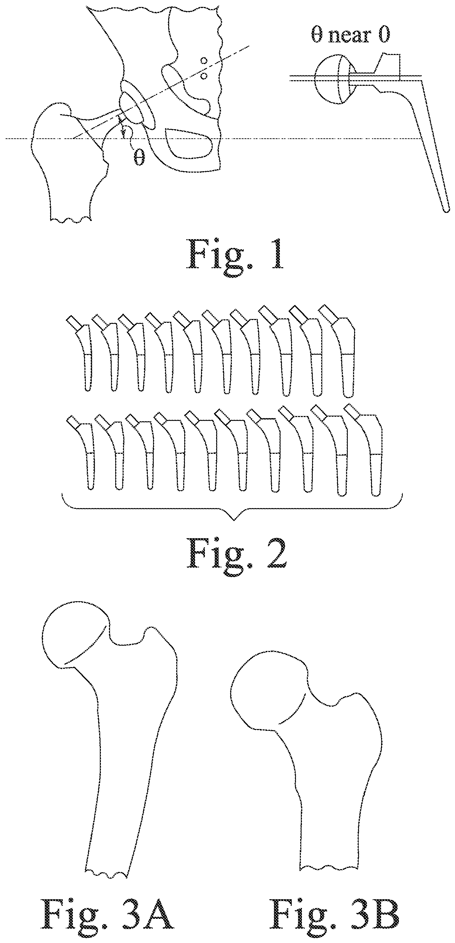

After a THA surgery, the ligamentous support structure has been compromised. Therefore, after implantation of a hip prosthesis, the natural anatomical structure is no longer in place and cannot resist out of plane forces and moments that may unseat the femoral head from the acetabular cup. As was stated previously, all present day THA implants attempt to maintain a femoral neck angle similar to the anatomical neck (see FIGS. 1 and 2).

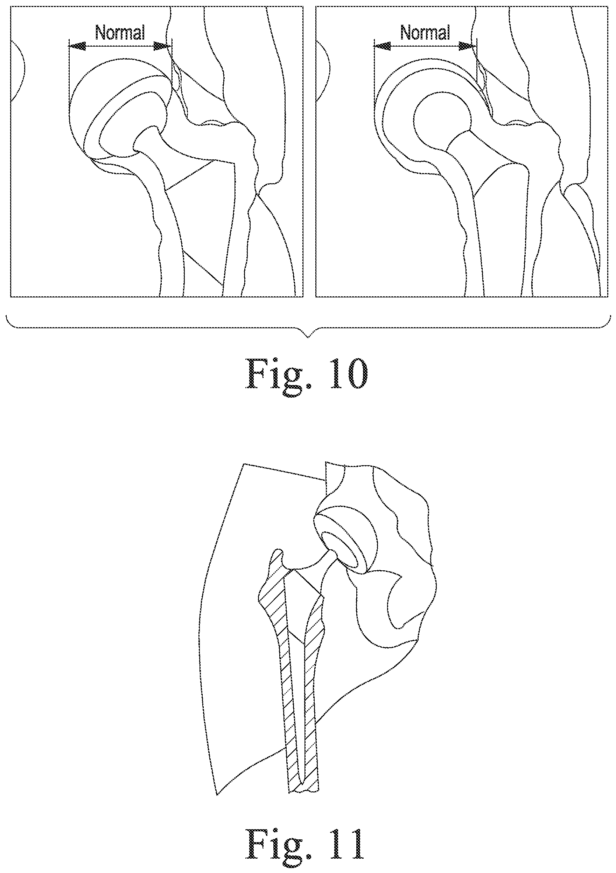

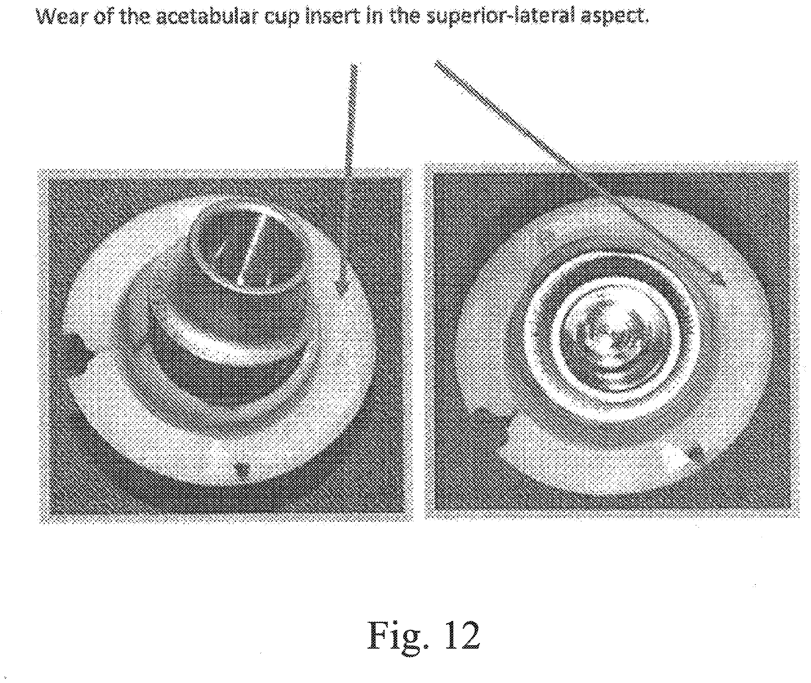

Referring to FIG. 10, superimposing a THA implant on the anatomical hip joint in FIG. 8, it can be seen that the femoral head contact area has been significantly reduced and when the orientation of the implant components are aligned, the hip joint is in a compromising state, so that both dislocation and femoral head separation are both not only possible, but possibly influenced to occur. The pull-out direction of the hip is aligned with the neck angle, which in present day implants is either perpendicular to the mechanical axis or close to perpendicular. This significant reduction of bearing surface contact area is a chief concern in present day THA, as the femoral head may also slide in the lateral direction, leading to an even more decreased bearing surface contact area, and polyethylene wear in the superolateral aspect (see FIG. 12). Therefore, in this orientation as the bearing force of the upper body is applied onto an implanted femoral head, which is aligned along the anatomical neck axis as in FIG. 10, this applied force may contribute to the femoral head either dislocating or separating from the acetabular cup. This phenomenon may be caused the inducement of a moment or introduction of a shear force along the femoral neck axis, pushing the femoral head out of the acetabular cup.

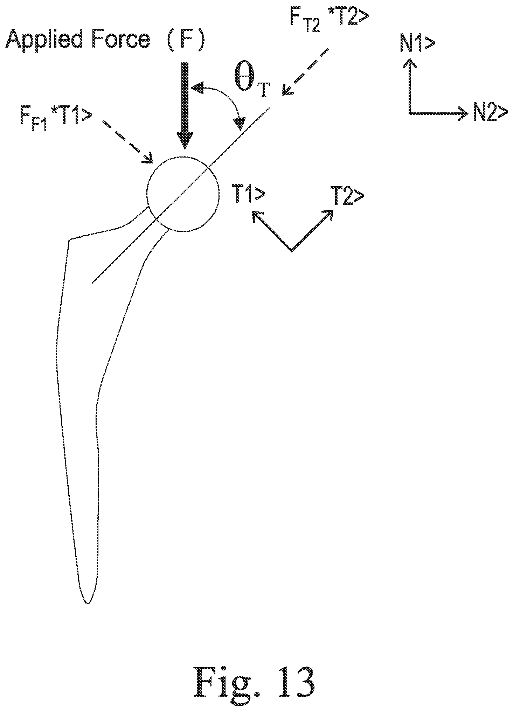

Referring to FIG. 13, another ongoing concern with THA is the failure associated with the trunnion and wear exhibited between the trunnion and a releasable femoral ball. Many, if not all, femoral implants used with THA include a femoral stem received within the intercondylar channel of a patient's femur, an integrated femoral neck, and a femoral head that is removable from an end (i.e., trunnion) of the femoral neck and is adapted to engage an acetabular cup.

Current femoral implants have been designed so that the femoral neck angle is aligned with the anatomical femoral neck angle. By doing so, the applied force of a patient's upper body (via the pelvis) onto the femoral implant head is at a significant angle with respect to the femoral neck angle. This applied upper body vertical force (F) acts at the hip joint as applied to a Newtonian reference frame along the N1> axis. This vertical force (F) is then broken into component forces in the trunnion reference frame (T1>, T2>, T3>). The relative transformation from the Newtonian reference frame to the trunnion reference frame is related to three sequential rotations, but the main rotation is denoted as θ_{T}. Using this rotation, the component forces are then defined as FT1 *T1> and FT2*T2>. The primary force of concern is FT1, which is applied in the TI> direction and throughout motion this force is actually attempting to unseat the ball from the trunnion.

During hip motion, the primary force FT1 is applied in the vertical direction, this can cause the femoral head to induce a micro-rocking motion between the trunnion of the femoral neck. This micro-rocking motion may also be induced by moments applied by this upper body force on the femoral head, with respect to the trunnion. The result of this micro motion is friction between the outer surface of the trunnion and the inner surface of the femoral head, causing wear between the bearing surfaces of the trunnion and the femoral head. In addition, this wear may cause particulate debris from the bearing surfaces that is distributed throughout a patient's body.

### INTRODUCTION TO THE INVENTION

The present invention is defined in claim 1 and is directed to components that may be implanted as part of a prosthetic joint such as a hip joint.

### BRIEF DESCRIPTION OF THE DRAWINGS

FIG. 1 is a pair of profile views showing prior art femoral components, with the left side image showing a neck design that is angled between 30-45 degrees with respect to a horizontal axis, while the right side image shows a neck design that is parallel to the same horizontal axis.
FIG. 2 is a profile view of a plurality of femoral components, without a femoral ball, to show the various orientations between 30-45 degrees of the femoral neck with respect to a horizontal axis.
FIG. 3 is a pair of profile views of a human proximal femur showing the native femoral ball.
FIG. 4 is a series of X-ray images showing femoral head dislocation from an acetabular cup.
FIG. 5 is a pair of fluoroscopic images of a THA implant, where the left side image shows the femoral head fully seated within the acetabular cup, while the right side image shows the femoral head separated or pulled away from the acetabular cup.
FIG. 6 is a pair of fluoroscopic images of a THA implant, where the left side image shows the femoral head fully seated within the acetabular cup, while the right side image shows the femoral head after it has slid out of the acetabular cup.
FIG. 7 is a human skeletal model showing the mechanical axis extending between the femoral head and the ankle joint, and passing through the knee joint.
FIG. 8 is a magnetic resonance image of a normal hip joint depicting the large contact area between the femoral head and the acetabulum.
FIG. 9 is a drawing showing the ligamentous supporting structure of a human hip joint.
FIG. 10 is a pair of images superimposing THA implants upon a natural, human hip joint to showing the decrease in bearing surface between the two.
FIG. 11 is a drawing of a THA implant showing the orientation of the components with respect to a native femur and native pelvis.
FIG. 12 is a pair of pictures showing two acetabular components taken from patients, where the components both show wear in the superior-lateral aspect.
FIG. 13 is a schematic of an exemplary femoral component depicting the applied forces on the trunnion.

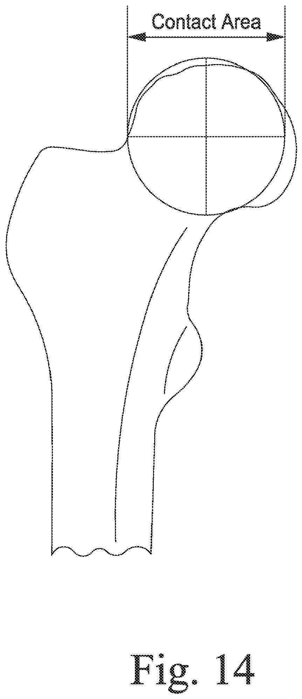

FIG. 14 is a profile view of a human proximal femur showing a superimposed sphere depicting the anatomical center of the femoral head.

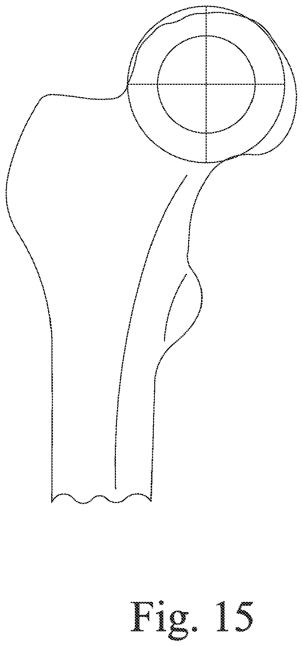

FIG. 15 is a profile view of a human proximal femur showing a superimposed sphere depicting the anatomical center of the femoral head and a superimposed, smaller sphere depicting the prosthetic center of the femoral ball component of a THA implant.

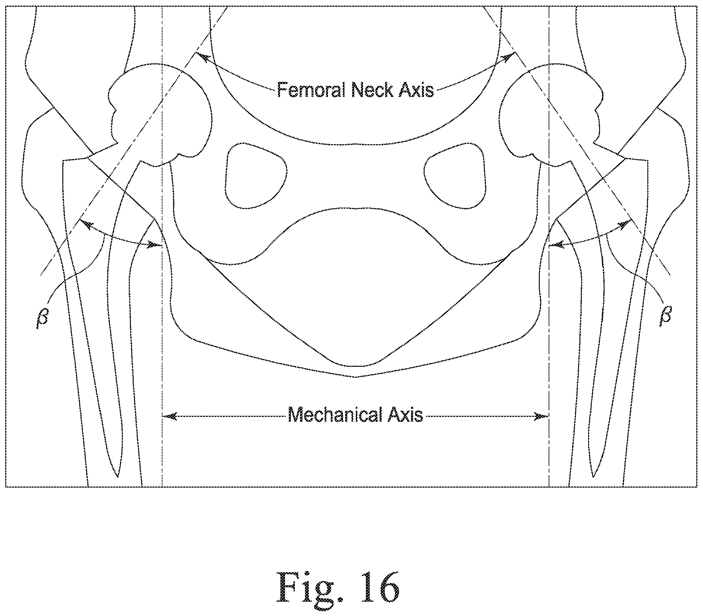

FIG. 16 is an X-ray image depicting the neck angle β (with respect to the mechanical axis) of a present day femoral components used in a THA, which is typically between 40-90 degrees.

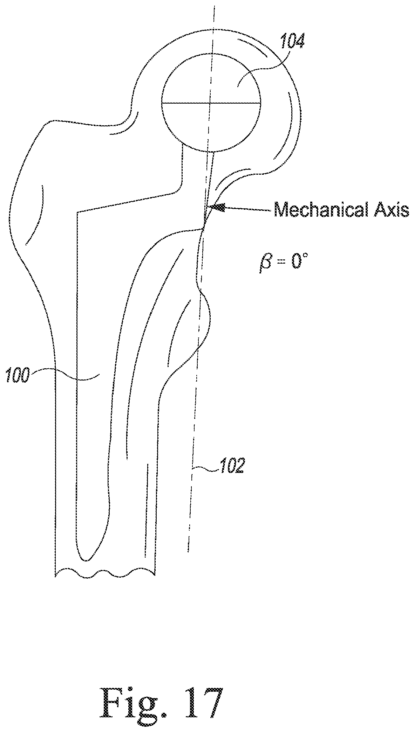

FIG. 17 is a profile view of a natural femur having an exemplary embodiment of the present disclosure superimposed thereon, where the neck angle β (with respect to the mechanical axis) is zero degrees.

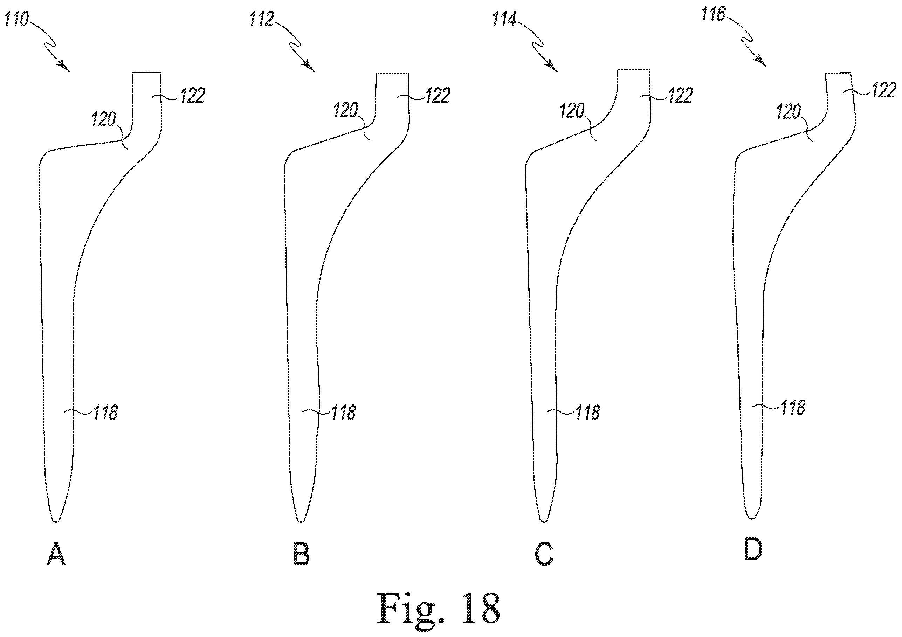

FIG. 18 is a profile view of a plurality of exemplary femoral components in accordance with the instance disclosure, showing variations in neck thickness, neck curvature, and overall neck shape.

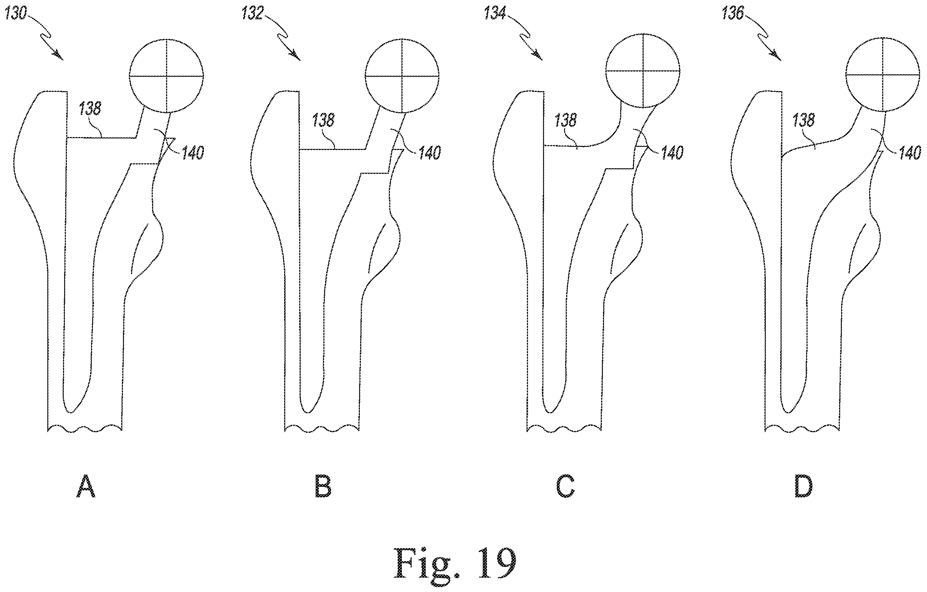

FIG. 19 is a profile view of natural femurs each having an exemplary embodiment of the present disclosure superimposed thereon, where variations in the neck shape are shown, such as where the neck is perpendicular to the femoral stem (A), or where the neck is concave on the medial or lateral side (B), or where the neck is concave on both the medial and lateral sides (C), or where the neck is curved.

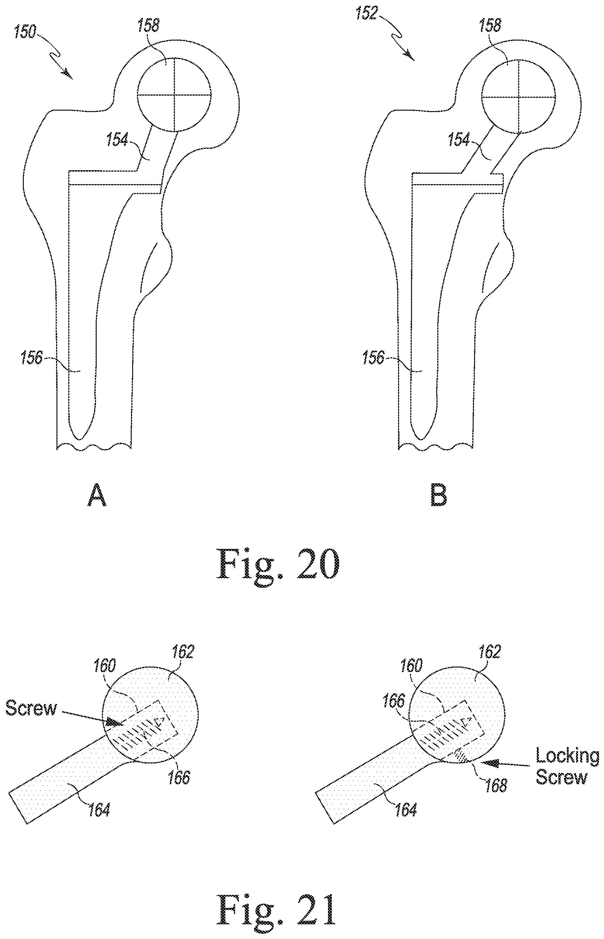

FIG. 20 is a profile view of natural femurs each having an exemplary embodiment of the present disclosure superimposed thereon, where the femoral component is modular to allow for various neck angles (A), and to allow for various neck positions with respect to the femoral stem (B).
FIG. 21 is an exemplary multi component neck and femoral ball inhibited from rotating with one another via a set screw.

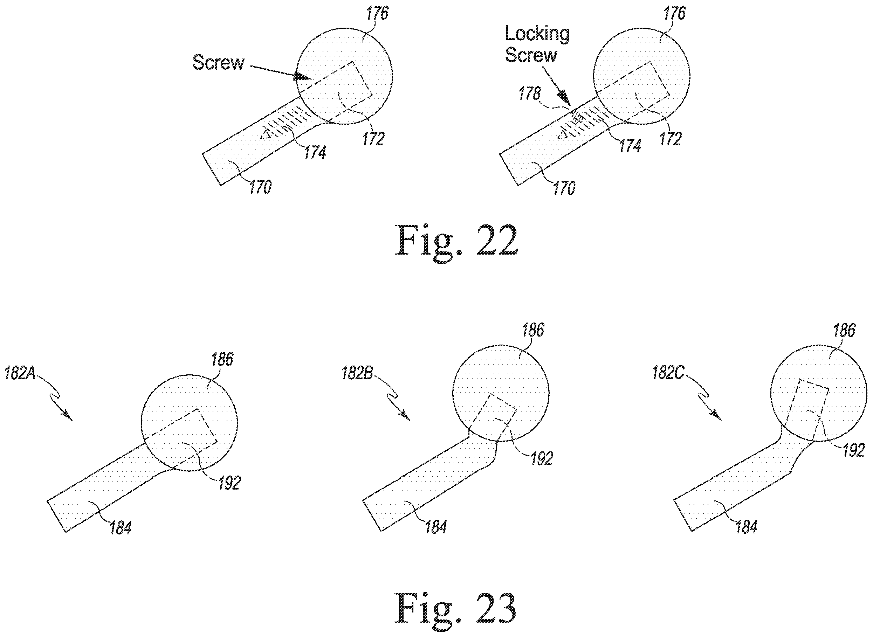

FIG. 22 is another exemplary multi component neck and femoral ball inhibited from rotating with one another via a set screw.
FIG. 23 is a series of integrated femoral necks and balls, where the angle of the trunnion axis differs with respect to that of the femoral neck axis.

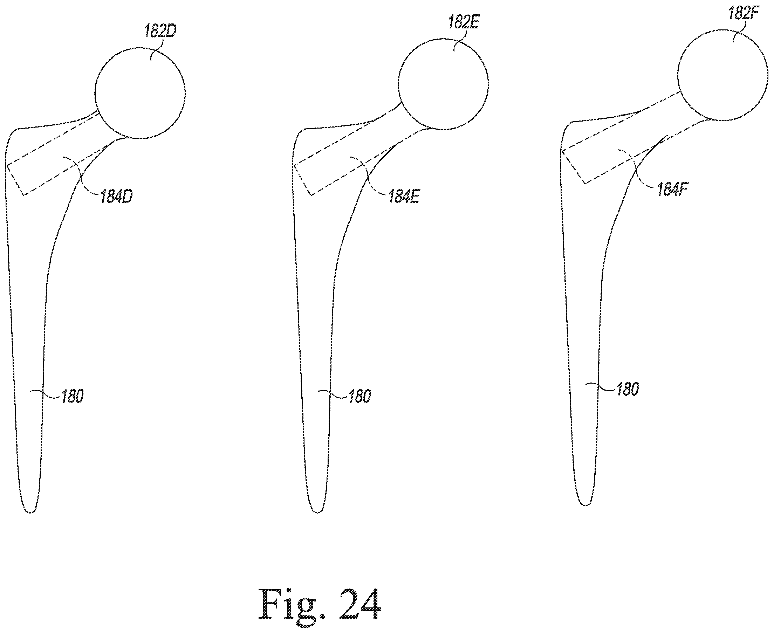

FIG. 24 is a series of exemplary femoral stems having inserted therein integrated femoral neck and ball components, where the necks have varying lengths.

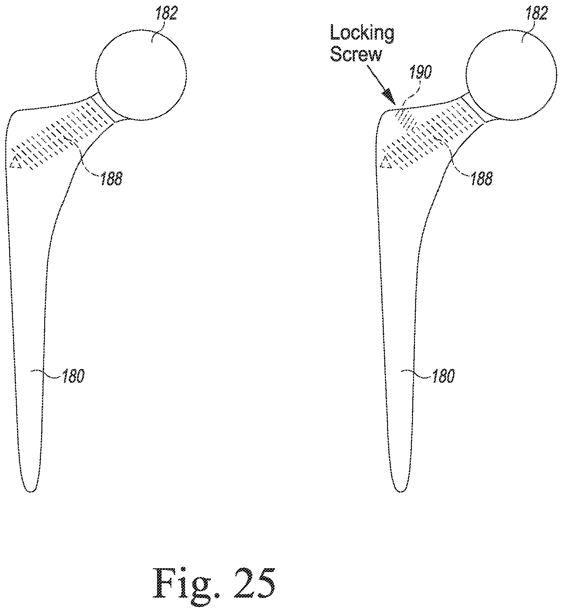

FIG. 25 is an exemplary femoral components shown with a stem having a pair of openings, at least one of which receives a set screw to inhibit rotation of an integrated femoral neck and ball with respect to the femoral stem.

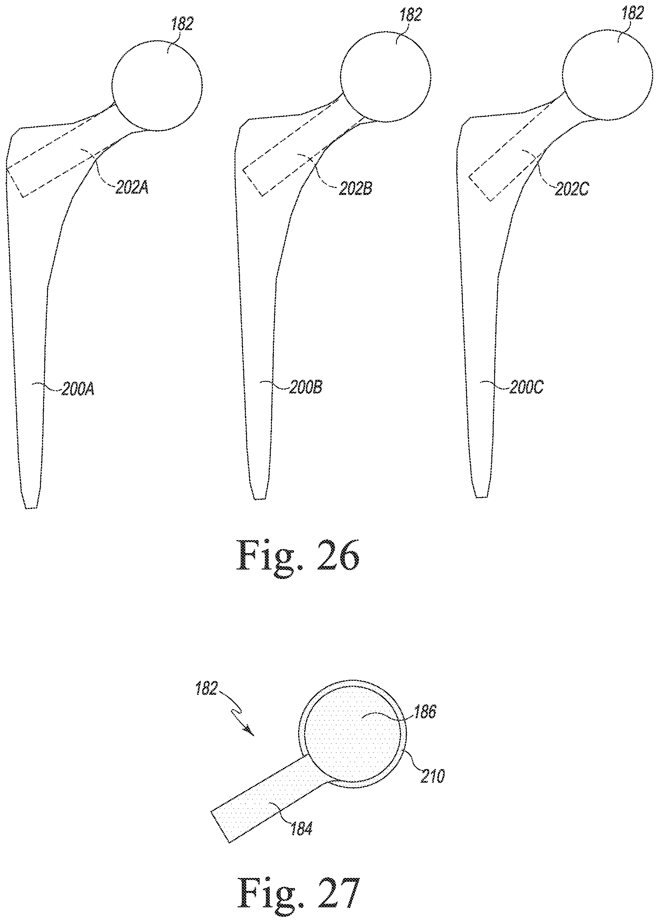

FIG. 26 is a series of exemplary femoral stems having cavities at various angles depending upon the angle desired between the femoral stem and an integrated femoral neck and ball component.
FIG. 27 is an exemplary integrated femoral neck and ball that includes a bearing surface cover.

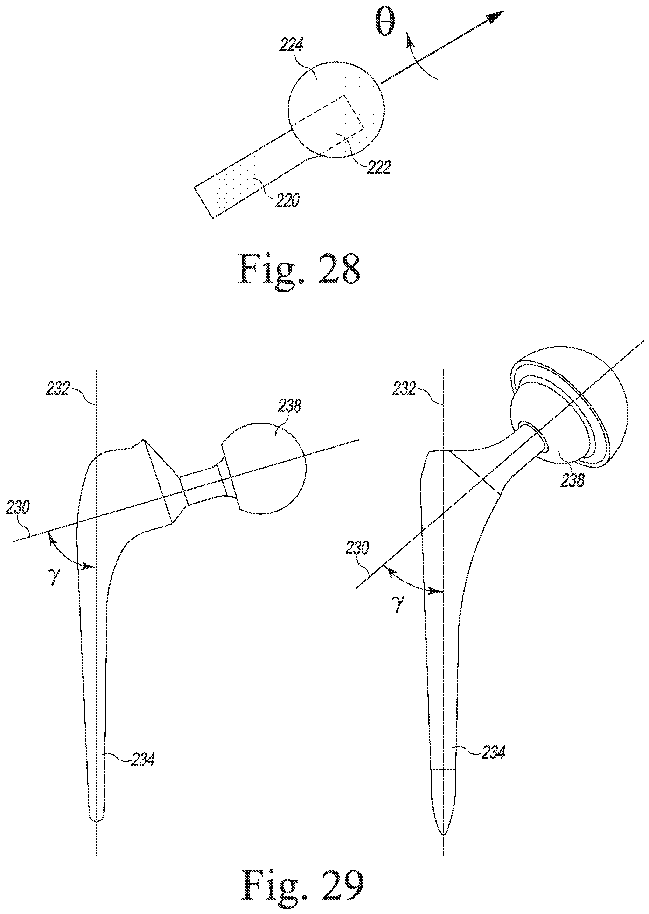

FIG. 28 is a mobile bearing femoral neck coupled to a ball component that allows the ball to rotate with respect to the neck.
FIG. 29 is a pair of present day femoral components shown with longitudinal axes for the stem and neck.

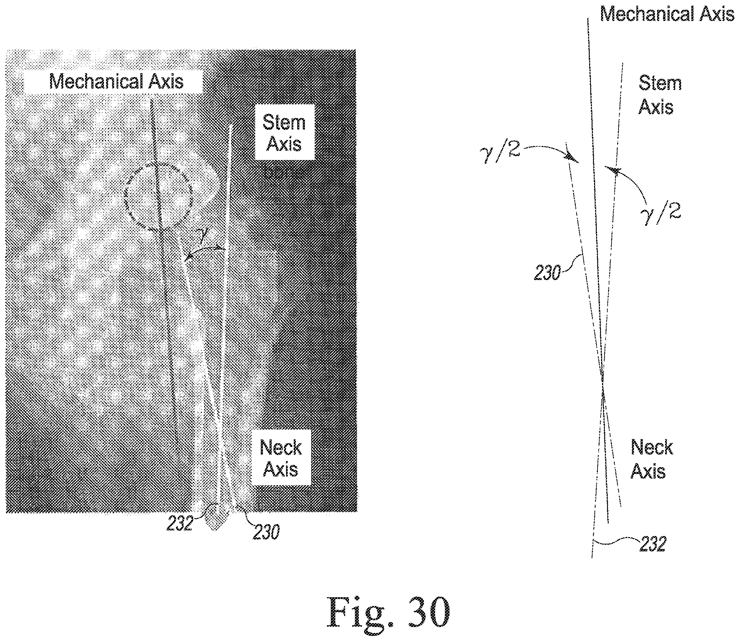

FIG. 30 is an X-ray superimposed with an exemplary acetabular component and femoral component in accordance with the instant disclosure, as well as a separate depiction of the relevant axes involved in the X-ray.

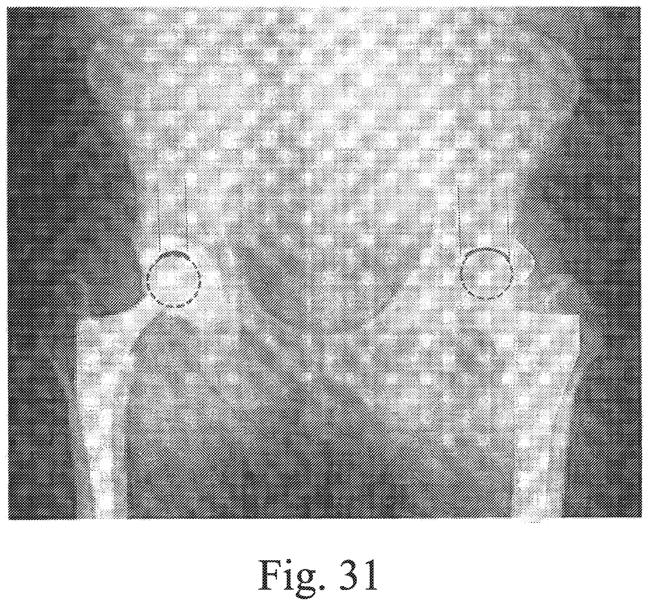

FIG. 31 is an X-ray showing on the left side a present day THA implant and on the right side a superimposed exemplary THA implant in accordance with the instant disclosure.

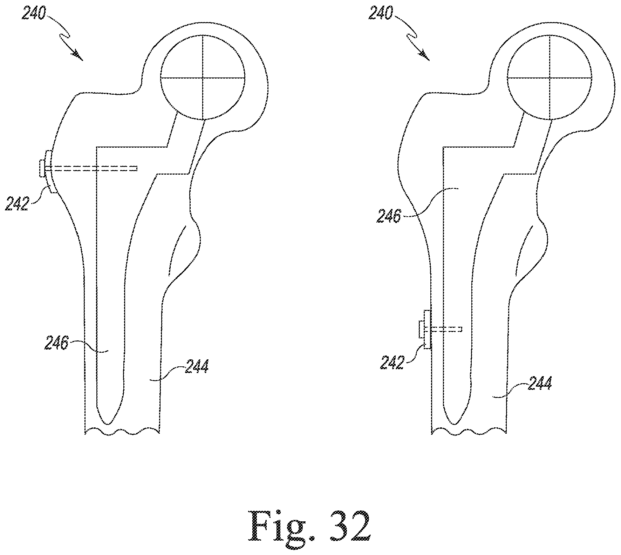

FIG. 32 is a cross-sectional view, from the side, of an exemplary femoral component having a reinforcement plate mounted at two different, exemplary locations.

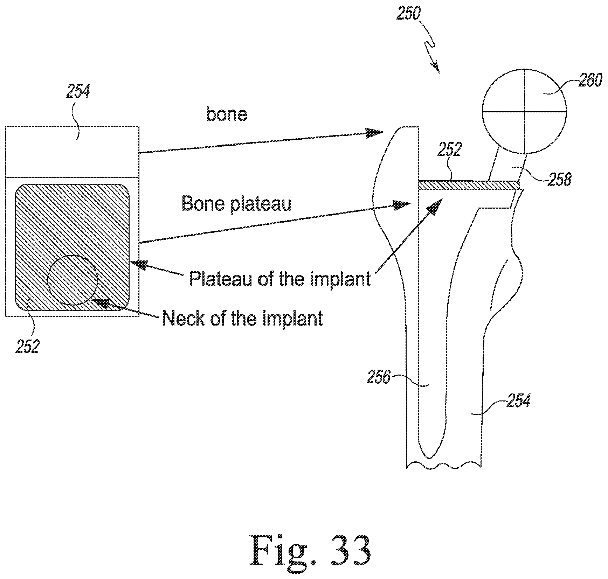

FIG. 33 is an exemplary femoral component in accordance with the instant disclosure.

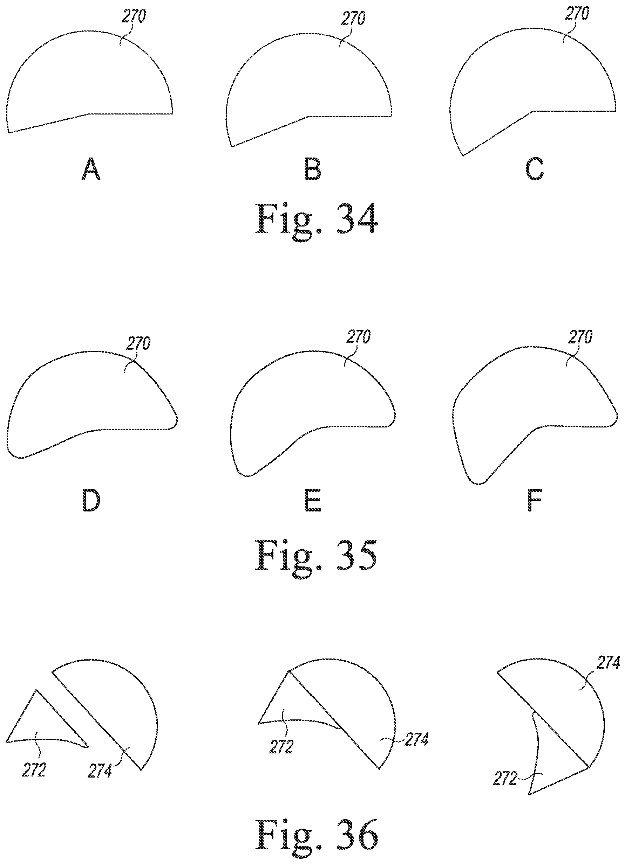

FIG. 34 is a first series of exemplary acetabular cups in accordance with the instant disclosure.
FIG. 35 is a second series of exemplary acetabular cups.
FIG. 36 is a first series of exemplary modular acetabular cups.

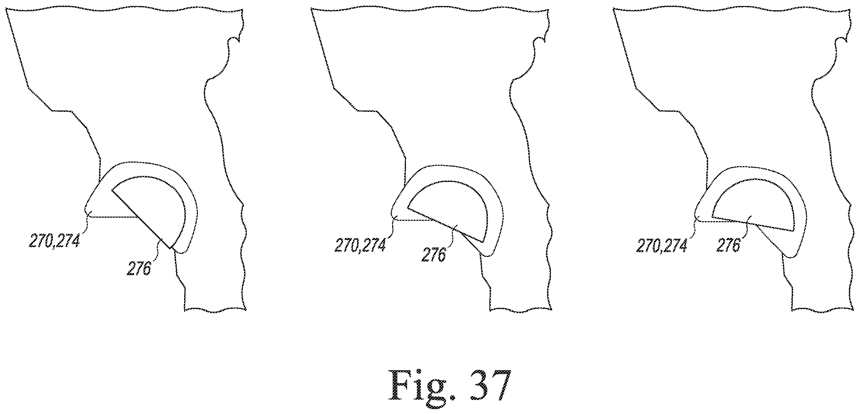

FIG. 37 is a series of profile cross-sections showing how exemplary acetabular liners would be oriented with respect to exemplary acetabular cups.

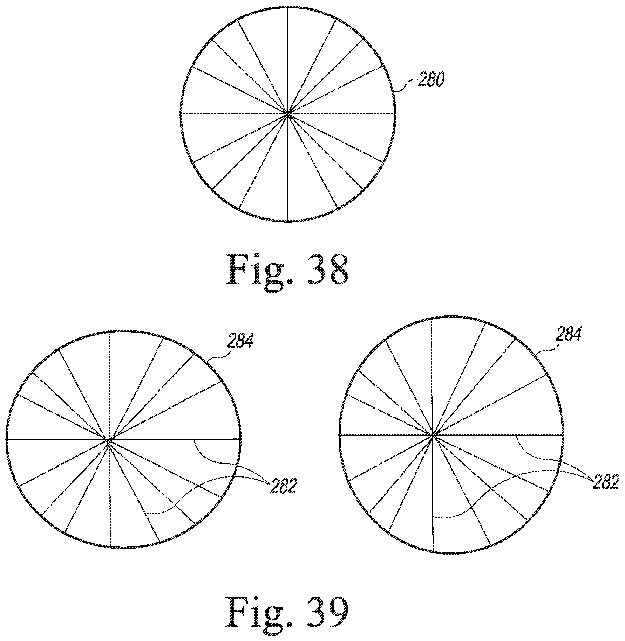

FIG. 38 is a depiction representing a spherical present day femoral head.
FIG. 39 is a pair of depictions representing, non-spherical exemplary femoral heads in accordance with the instant disclosure.

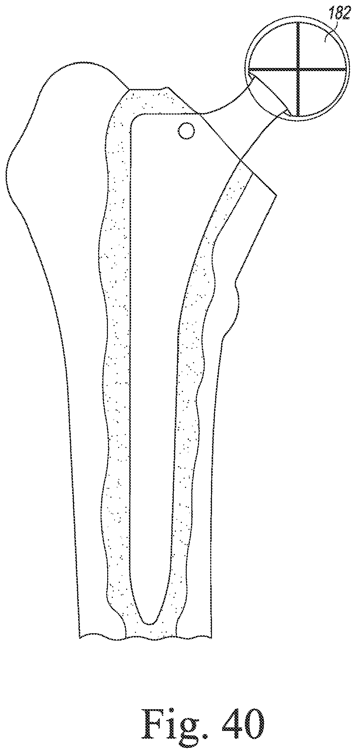

FIG. 40 is an exemplary femoral component incorporating a non-spherical femoral head.

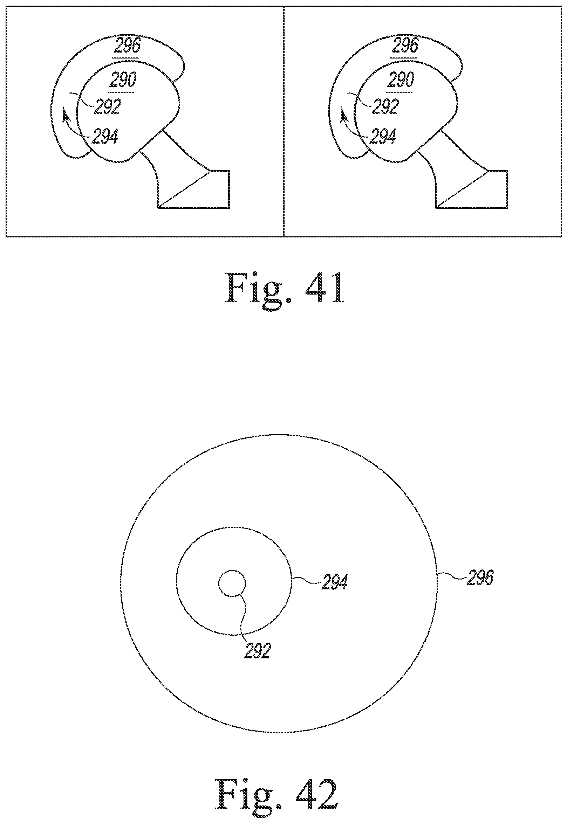

FIG. 41 is a pair of cross-sectional views showing an exemplary THA implant that incorporates a head projection and a liner recess.
FIG. 42 is an overhead view of the liner of FIG. 41, showing the outline the projection would occupy if present.

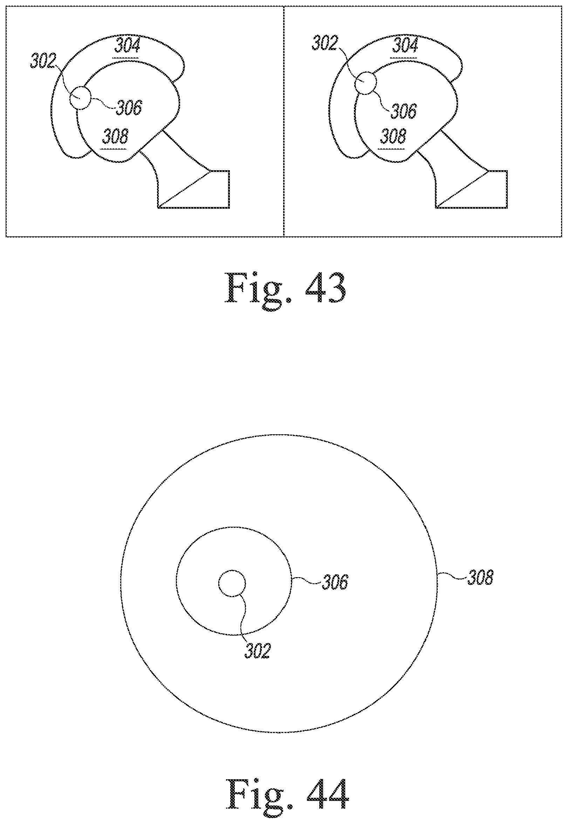

FIG. 43 is a pair of cross-sectional views showing an exemplary THA implant that incorporates a head recess and a liner projection.
FIG. 44 is an overhead view of the liner of FIG. 43, showing the outline the recess would occupy if present.

### DETAILED DESCRIPTION

The exemplary embodiments of the present invention are described and illustrated below to encompass components that may be implanted as part of a prosthetic joint, such as a hip joint. Of course, it will be apparent to those of ordinary skill in the art that the embodiments discussed below are exemplary in nature and may be reconfigured without departing from the scope of the present invention as defined by the claims.

This present disclosure allows for the bearing surface force of the hip joint, along the mechanical axis of the leg, to pass through the implanted femoral component, which may lead to one or more of the following: (1) an increased bearing surface contact area; (2) a significant reduction in bearing surface contact stress; (3) a significant reduction in femoral head separation; and, (4) a reduction of the out-of-plane forces and bending moments applied to the trunnion. At the same time, the implanted femoral component may attempt to maintain the concentricity of the anatomical hip joint.

Ideally, an implanted femoral component and acetabular component should be the same size, shape, and in the same orientation as the native joint components that each prosthetic component replaces. Unfortunately, no hip implant is designed with all three of these features for various reasons. One of the primary reasons is that the anatomical shape of each patient's femoral head and acetabulum are quite different (i.e., unique). Also, it is difficult to design an acetabular cup and femoral head that are anatomical in shape because fixation of the acetabular cup may be more difficult and proper preparation of the acetabulum even more difficult. Recent attempts at developing resurfacing prostheses that maintain the size of the femoral head have been designed, but later post-operative follow-up results have not been acceptable and many of these prostheses have been recalled from the market. Therefore, present day hip joint implants (including femoral and acetabular components) have been designed to maintain the anatomical orientation of the femoral neck, without realizing that a reduced femoral head size will lead to a decreased contact area and increased contact stress.

The present disclosure is made in the context of considerations relevant to maintaining or very closely approximating the native anatomical spheres of the femoral head and acetabular cup (see FIG. 14). By maintaining the anatomical concentric spheres when designing and positioning THA implants, as shown in FIG. 15, the mechanical axis passes through the femoral head of the implant. Therefore, even though the implanted femoral head size may be reduced, compared to the anatomical femoral head, it is ideal for the spherical center of the implanted femoral head to be in the same location as the anatomical spherical center.

Some of the exemplary femoral implants of the present disclosure include a femoral neck longitudinal axis that is parallel or near parallel to the mechanical axis of the human leg. Present day femoral implants have attempted to align the femoral neck with the anatomical neck, creating an increased angle, (β, with respect to the mechanical axis of the human leg (see FIG. 16). When the implanted cup is oriented at ninety degrees with respect to the femoral neck angle β, this allows for femoral head separation and dislocation to occur. Also, the weight-bearing force is not applied through the neck of the femoral implant, leading to transverse forces being applied through the femoral head into the trunnion of the femoral implant and leading to induced moments that cause failure and/or loosening between the trunnion and femoral head.

Referring to FIG. 17, in an exemplary femoral implant 100, the femoral neck axis is either parallel with respect to the mechanical axis 102 or is angled, β, less than twenty-five degrees with respect to the mechanical axis. Having the angle β be less than twenty-five degrees with respect to the mechanical axis is operative to resist dislocation of the femoral head 104 from the acetabular cup. In present day THA implants, the bearing surface force is applied through the hip joint along the mechanical axis tending to move joint has been sacrificed, there are not any forces resisting the femoral head from sliding out of the acetabular cup. In this exemplary femoral implant 100, however, the bearing surface force is applied through and along the mechanical axis 102 to drive the femoral head 106 toward the femoral cup. Therefore, during weight-bearing activity, the femoral head 106 may only separate from the acetabular cup through the neck axis. In an instance where a load is applied to the hip joint, it is virtually impossible for the femoral head 106 to pull away from the acetabular cup along the femoral neck axis.

Referring to FIG. 18, alternate exemplary femoral implants 110, 112, 114, 116 each include a one-piece femoral stem 118 and neck 120 that includes a trunnion 122 to which is mounted a separable femoral head (not shown). In this exemplary embodiment, the femoral stem 118 includes a longitudinal axis that is either parallel with or angled within twenty degrees of a longitudinal axis of the neck 120. As discussed above, an ideal orientation is for the longitudinal axes of the neck 120 and stem 118 to be in parallel. The greater the angle between the longitudinal axes, the greater the chance of rotation between the trunnion 122 and the separable femoral head. By reducing the angle between the axes, the femoral head is secured to the trunnion 122 predominantly in the direction of the applied bearing surface force vector on or near the mechanical axis. In this manner, under loaded conditions, the femoral head is compressed along the longitudinal axis without applying out-of-plane forces to the trunnion 122 or inducing significant moments that cause micro-motion between the femoral head and trunnion. No matter what shape and contour differences exist, the common orientation between the exemplary femoral implants depicted in FIG. 18 is a twenty degree or less deviation between longitudinal axes of the femoral stem 118 and neck 120 (specifically, the trunnion 122).

Referring to FIG. 19, still further exemplary femoral implants 130, 132, 134, 136 include a perpendicular shelf 138 from which the femoral neck 140 extends. The exemplary femoral implants 130, 132, 134, 136 may have a perpendicular shelf on the inferior side of the stem, whereas the superior femoral stem is angled between zero and thirty degrees with respect to the mechanical axis. The medial, lateral, anterior or posterior aspect of the femoral stem 130, 132 may be of irregular shape, such as concave allowing for greater defined rotation or rotations to occur. All aspects of the femoral implant 134 may be irregular in shape, such as concave similar to an hour glass, or the femoral implant 136 may be curved and the thickness of the stem may vary across a horizontal plane.

Referencing FIG. 20, an exemplary femoral implant 150, 152 may be modular so that upper and lower portions of the femoral stem 156 are mounted to one another allow for variable neck lengths, angles, and shapes, as well as femoral heads that are angled and offset from the femoral neck, For example, the first exemplary femoral implant 150 includes an upper femoral stem having a neck 154 significantly spaced apart from the femoral stem 156, where the angle of the femoral stem and the femoral neck are substantially parallel. The second exemplary femoral implant 152 also includes a two piece femoral stem, but this time the upper portion of the stem 156 includes a neck 154 that is spaced apart from the lower portion of the femoral stem less than the first exemplary femoral implant 150, but the neck is angled approximately fifteen degrees with respect to the lower femoral stem. Accordingly, it should be understood that by using modular femoral implants, one can easily provide for interchangeable upper femoral stem portions that change the position and orientation of the femoral neck (an ultimately the femoral ball 158) with respect to the lower femoral stem portion where the longitudinal axis is established.

Referring back to FIG. 13, an ideal angle for θ_{T} is zero. When θ_{T} is zero, the applied force vector F is coaxial with the longitudinal axis of the trunnion and femoral head. In other words, when θ_{T} is zero, there are no rotational force introduced from the applied force vector F that would tend to rotate the femoral head (or leave stationary if rotation of the trunnion occurs) in a first direction and rotate the trunnion (or leave stationary if rotation of the femoral head occurs) in a second direction. As discussed previously, it is the presence of rotational force(s) that causes micro motion between the trunnion and the femoral head.

FIG. 21 depicts an instance where θ_{T} is not zero. Even if one were to use such an angle between the longitudinal axis of the femoral stem and femoral neck, the present disclosure provides a number of exemplary solutions to retard micro motion between a trunnion 160 and femoral head 162. In exemplary form, a femoral implant includes a femoral stem (not shown) and a femoral neck 164 angled roughly forty-five degrees with respect to the longitudinal axis of the femoral stem. At the end of the femoral neck is a trunnion 160 having a threaded end 166 that is adapted to be received within a primary threaded cavity of a femoral head 162. The femoral head 162 also includes a second threaded cavity that intersects the primary threaded cavity. A threaded connector 168 (e.g., a screw) is adapted to be received within the second threaded cavity and advanced within the second threaded cavity until coming into contact with a portion of the threaded end 166 of the trunnion 160. More specifically, the threaded connector 168 is tightened to inhibit rotation of the threaded end 166 with respect to the femoral head 162. It should be noted, however, that a threaded trunnion need not be utilized. For instance, the trunnion may have a non-circular shape (such as triangular or rectangular) that by itself retards rotation of the femoral head 162 with respect to the trunnion 160.

Referring to FIG. 22, another exemplary femoral implant includes a femoral stem (not shown) and a femoral neck 170 angled roughly forty-five degrees with respect to the longitudinal axis of the femoral stem. At the end of the femoral neck is a trunnion 172 having a threaded cavity that is adapted to receive a threaded end 174 extending from a femoral head 176. The neck 170 of the femoral implant also includes a second threaded cavity that intersects the primary threaded cavity. A threaded connector 178 (e.g., a screw) is adapted to be received within the second threaded cavity and advanced within the second threaded cavity until coming into contact with a portion of the threaded end 174 of the femoral head 176. More specifically, the threaded connector 178 is tightened to inhibit rotation of the threaded end 174 with respect to the femoral neck 170. It should be noted, however, that a threaded femoral ball 176 need not be utilized. For instance, the end 174 of the femoral ball 176 may have a non-circular shape (such as triangular or rectangular) to fit within a corresponding shaped cavity of the femoral neck 170 so that the interaction between the end 174 and the femoral neck 170 by itself retards rotation of the femoral head 176 with respect to the trunnion 172.

Referring to FIGS. 23-25, yet another exemplary femoral implant includes a femoral stem 180 that is removably coupled to a one-piece femoral neck and femoral head implant 182. The femoral neck 184 of this one-piece implant 182 may take on any number of shapes and sizes (i.e., may vary thickness, orientation, cross-section, etc.). By creating a one-piece device and seamless connection between the femoral neck 184 and femoral head 186, rotation between these two elements is inhibited. And the one-piece implant 182 is adapted to be inserted in a cavity of a corresponding femoral stem 180 to comprise a complete femoral implant.

In exemplary form, referring again to FIG. 25, the femoral stem 180 includes a threaded cavity that is adapted to receive a threaded end 188 extending from an integrated femoral neck and ball 182. The stem 180 of the femoral implant also includes a second threaded cavity that intersects the primary threaded cavity. A threaded connector 190 (e.g., a screw) is adapted to be received within the second threaded cavity and advanced within the second threaded cavity until coming into contact with a portion of the threaded end 188 of the integrated femoral neck and ball 182. More specifically, the threaded connector 190 is tightened to inhibit rotation of the integrated femoral neck and ball 182 with respect to the femoral stem 180. It should be noted, however, that a threaded end 188 need not be utilized. For instance, the end 188 of the femoral neck and ball 182 may have a non-circular shape (such as triangular or rectangular) to fit within a corresponding shaped cavity of the femoral stem 180 so that the interaction between the end 188 and the femoral stem 180 by itself retards rotation of the femoral neck and ball 182 with respect to the femoral stem 180.

As shown in FIG. 23, exemplary one-piece femoral neck and head implants 182 may provide femoral head 186 offset from or aligned with the femoral neck 184. In exemplary form, a first one-piece femoral neck and femoral head implant 182A provides the neck 184 and center of the head 186 aligned along a longitudinal axis. But the second and third one-piece femoral neck and femoral head implants 182B, 182C include head 186 centers that are offset from a longitudinal axis extending through the femoral neck 184. In addition to being offset from the longitudinal axis, the second and third one-piece femoral neck and femoral head implants 182B, 182C include femoral heads 186 that are angled differently with respect to the femoral neck 184. In particular, the second one-piece femoral neck and femoral head implant 182B includes a trunnion 192 angled approximately fifty degrees with respect to the longitudinal axis extending through the femoral neck 184, while the second one-piece femoral neck and femoral head implant 182C includes a trunnion 192 angled approximately seventy degrees with respect to the longitudinal axis of the femoral neck 184. In this manner, the one-piece femoral neck and femoral head implants 182A, 182B, 182C may include trunnions 192 that operate to angle the femoral head 186 with respect to the femoral neck 184 anywhere between zero and ninety degrees and offset the femoral head with respect to the femoral neck anywhere between 1 to 40 millimeters.

As shown in FIG. 24, exemplary one-piece femoral neck and femoral head implants 182 may also include necks 184 having different lengths. In exemplary form, a first one-piece implant 182D includes a femoral neck 184D having a shaft with a generally cylindrical shape (i.e., longitudinal circular cross-section) that is adapted to be received within a corresponding cavity of a femoral stem 180. In this exemplary embodiment, the corresponding cavity is also cylindrical. But those skilled in the art will understand that the femoral neck shaft may take on any number of cross-sections such as, without limitation, cross-sections having three or more sides. Likewise, the corresponding cavity within the femoral stem will be sized to mate with and accommodate whatever cross-section the femoral neck 184 embodies. By providing necks with differing lengths, the surgeon is able to choose the appropriate one-piece implant based upon the patient's preferred anatomical spacing between the acetabular cup and femoral stem. In this exemplary embodiment, the first one-piece implant 182D includes a femoral neck 184D having a length of 5 to 10 mm, the second one-piece implant 182E includes a femoral neck 184E having a length of 11 to 30 mm, and the third one-piece implant 182F includes a femoral neck 184F having a length of 31 to 80 mm.

Referring to FIG. 26, it is also within the scope of the disclosure to provide femoral stems/shafts 200A, 200B, 200C that are adapted to orient the one-piece implants 182 at different angles. For example, as shown, a first exemplary femoral stem 200A includes a cavity that receives a one-piece neck and ball implant 182. In this discussion, for purposes of explanation only, the one-piece neck and ball implant 182 is the same as discussed previously. But those skilled in the art will understand that various one-piece neck and ball implants may be used with the exemplary femoral stems 200A, 200B, 200C, such as any one of those discussed previously or hereafter.

The first exemplary femoral stem 200A includes a cavity 202A having a longitudinal axis angled approximately forty-five degrees with respect to a longitudinal axis of the femoral stem. In contrast, the second exemplary femoral stem 200B includes a cavity 202B having a longitudinal axis angled approximately fifty-five degrees with respect to a longitudinal axis of the femoral stem. Finally, a third exemplary femoral stem 200C includes a cavity 202C having a longitudinal axis angled approximately sixty-five degrees with respect to a longitudinal axis of the femoral stem. Those skilled in the art will realize that cavities may be formed into the femoral stem at various angles to orient the one-piece neck and head implant 182 at any angle between twenty and ninety degrees with respect to the longitudinal axis of the femoral stem.

Referencing FIG. 27, it is also within the scope of the disclosure to attach a low bearing surface 210 to the exterior of the femoral head 186. By way of example, the one-piece neck and head implant 182 may be fabricated from metals, polymers, ceramics, and composites. Because fabrication of the one-piece neck and head implant 182 provides an integrated structure, it may be advantageous to change the bearing surface by attaching a material 210 to the exterior of the femoral head 186. In this exemplary embodiment, the one-piece neck and head 182 are fabricated from a metal or metal alloy. But end implant is to include a femoral head bearing surface 210 that comprises another material, such as high density polyethylene. In order to mount the new bearing surface 210 to the femoral head 186, one may utilize compression molding, hydro-dynamically attachment, or other surface fixation technology. One of the advantages or this approach is that the interface surfaces (i.e., interior of new bearing surface 210 and exterior of femoral head 186) have much more surface area (in comparison to the typical surface area between the trunnion and the inside of the femoral head) available in order to attach the new bearing surface 210 to the femoral head 186.

Using a one-piece femoral head and neck implant 182 addresses the potential for micro-rocking motion between the femoral head 186 and femoral neck 184. But the one-piece femoral head and neck implant 182 also potentially addresses other issues. As part of THA, those skilled in the art are familiar with a surgeon reaming a pelvis to prepare the pelvis to receive an implanted acetabular cup. If the surgeon reams/removes too much bone or reams at an incorrect angle, the one-piece head and neck implant allows the surgeon to correct for surgical preparation errors of this type and ensure that the femoral head is positioned in the anatomically correct position. Reference is had to U.S. Patent Application Serial No. 13/330,259, for a more detailed discussion of anatomically correct positioning of a femoral head using concentric centers of the femoral head and native acetabulum.

Referring to FIG. 28, an exemplary mobile bearing femoral implant includes a femoral stem (not shown) and a femoral neck 220, for example angled roughly forty-five degrees with respect to the longitudinal axis of the femoral stem, but this angle of orientation could be greater or lesser than forty-five degrees so that the femoral spherical center could be aligned with the anatomical spherical center. At the end of the femoral neck 220 is a trunnion 222 having a rounded, cylindrical end that is adapted to be received within a primary, smooth cavity of a femoral head 224. In this exemplary embodiment, the outer bearing surface of the rounded, cylindrical end of the trunnion 222 and the interior surface(s) delineating the smoother cavity of the femoral head 224 are highly polished to reduce friction therebetween. In particular, the outer bearing surface of the trunnion 222 is sized allow rotation of the trunnion with respect to the femoral head 224. Moreover, the trunnion 222 may be slightly tapered to allow the femoral head 224 to translate up and down with respect to the trunnion.

Referring to FIG. 29, although the ideal design of a femoral implant is to have the femoral neck axis 230 to be parallel with the mechanical axis of the leg, the next optimal orientation would be to minimize the angle (denoted as "γ") between the longitudinal axis 232 of the femoral stem 234 and the longitudinal axis 230 of the femoral neck 236. In present day THA, this angle γ, is quite large. When a force in the direction of gravity is applied to the femoral head 238, then an increased moment is applied, leading to greater forces in the muscle structures to balance the hip joint, leading an increased bearing surface force.

Referencing FIGS. 30 and 31, the instant disclosure proposes that the angle difference between these two axes 230, 232 be as small as possible. Having a small angular difference allows for a more optimal dispersion of forces through these structures and a reduction in moments at the hip joint with respect to the main bearing surface force that is applied along the mechanical axis.

As stated previously, a hip stem and neck with an optimal angle, γ, and optimal angles between the femoral neck axis with respect to the mechanical axis (γ/2) and the femoral stem axis and the mechanical axis (γ/2) will allow for optimal contact area between the femoral head and the acetabular cup (see FIG. 31). As shown in FIG. 31, the left side view is of a present day THA implant oriented with a rather large angle γ and a relatively small contact area between the femoral head and acetabular cup insert, while the right side view is an overlay of an exemplary embodiment of the instant disclosure showing an optimal angle γ and how this helps to increase the contact area between the femoral ball and acetabular cup.

Referring to FIG. 32, although loosening the femoral stem with respect to the native femur has been reduced to a lower incidence rate, it still remains a concern in implant longevity. Therefore, an exemplary femoral implant 240 may make use of a reinforcement plate 242 (and corresponding nail or screw) concurrently mounted to the femur 244 and femoral stem 246. This reinforcement plate 242 may be inserted at variable locations along the femoral stem 246 and operates to ensure that the femoral stem remains fixated to the femur 244, even during more dynamic, challenging activities.

Referencing FIG. 33, in order to resist out of plane forces and bending moments, an exemplary femoral component 250 includes an upper aspect 252 of a femoral stem that comprises a flat, rounded or anatomically shaped plate mounted to the contoured top of the femur 254. This plate 252 may be modular in nature and connected to the upper portion of the femoral stem 256 and include an integrated neck 258 and possibly an integrated femoral ball 260.

Referring to FIGS. 34 and .35, placement of the femoral neck along the mechanical axis allows the femoral head to maximize contact area and resist the femoral head from separating from the acetabular cup (see FIG. 31). But the shape of the acetabular cup may also be modified to assist in capturing the femoral head therein. For example, an exemplary femoral cup 270 includes an extended surface area to increase the potential contact area between the femoral head and acetabular cup insert across the range of motion of the femoral head. In particular, the intent is to maximize the contact area (between femoral head and acetabular cup (i.e., insert)) throughout the range of motion of the femoral head. Extending the surface area of the acetabular cup leads to maximal femoral head contact area and greater contact between the acetabular cup and residual bone surface. FIGS. 24 and 25 provide exemplary acetabular cup 270 shapes that increase the surface area beyond present day one hundred and eighty degree coverage.

It should also be noted that the exemplary acetabular cups 274 may include separable extensions 272 so that the final cup comprises multiple pieces, such as the exemplary cups shown in FIG. 36. In these exemplary cups 274, the extensions 272 provide up to forty-five degrees of additional surface area coverage.

Also, as shown in FIG. 37, the positioning of the acetabular cup 270, 274 and its liner cup 276 within acetabular cup could also be modular, depending on the placement of the acetabular cup within the acetabulum. Since the extension cup 270, 274 covers more than 180 degrees of surface area, it is possible to place the liner cup 276 either in a more vertical or horizontal orientation within the acetabular cup. The acetabular cup liner 276 could be 180 degrees and be positioned with the acetabular cup 270, 274 implanted having a surface area greater than 180 degrees. Therefore, depending on the position of the acetabular cup 270, 274 to maximum bone fixation area, the polyethylene liner 276 could be positioned and/or oriented in multiple ways within the acetabular cup, allowing for correlating the implant spherical center with the anatomical spherical center. The polyethylene liner 276 may also be of the exact same shape as the acetabular cup component 270, 274, having a surface area greater than 180 degrees.

One of the optional objectives of the previous mentioned implant designs is to orient the hip components to maximize the in vivo contact area, which in-turn will decrease the in vivo contact stresses. A more vertical positioned femoral neck also leads to resistance of dislocation and femoral head separation. If it is desirable to having a more traditional femoral implant with a femoral neck orientation similar to the anatomical neck, the femoral head shape may be altered to maximize contact area and in-turn decrease the potential for dislocation and femoral head separation.

Referring to FIGS. 38-40, present-day femoral head implants 280 are spherical in shape, comprising a round solid object having a circumferential surface with every point equidistant from its center. Unfortunately, this type of design, as explained previously in this patent, may induce hip dislocation and femoral head separation. One improved embodiment that may increase the femoral head 284 bearing surface contact area includes a bearing surface where not every surface point is equidistant from the femoral head center (see FIGS. 39 and 40). The variable radii 282 shown in FIGS. 39 and 40 may be incorporated throughout the femoral head 284 or in a covering on the femoral head comprising a bearing surface. The acetabular cup and the liner would also have an irregular shape that will accept the design change to maximize hip mechanics by increasing the contact area. Therefore, this re-designed shaped acetabular cup will be altered based on the mating femoral head. A more elongated femoral head will result in a more elongated acetabular cup and/or liner. The increased bearing surface contact area disperses the applied hip force through the increased area, thereby leading to lesser shear forces applied on the femoral head 284, consequently allowing the femoral head to increasingly resist dislocation and femoral head separation.

The exemplary femoral head 284 may also have a flatter variable radii 282 mating with the medial, inner aspect of the acetabular cup or the opposite may be incorporated in the design, so that the outer shape of the femoral head 284, mating with the inner aspect of the acetabular cup is more elliptical in shape.

Referring to FIGS. 41 and 42, in a further exemplary embodiment 288, a femoral head 290 includes a projection 292 that is received within a dedicated recess 294 of an acetabular cup liner 296. The projection 292 has a range of motion with respect to the dedicated recess 294 that allows for limited abduction and adduction of the patient's femur with respect to the pelvis. More specifically, as the femoral head 290 is rotated in a direction of abduction, the projection 292 moves closer to its range of motion boundary established by the dimension of the dedicated recess 294. When the femoral head 290 continues to be rotated in a direction of abduction, eventually the projection 292 will contact the edge (i.e., boundary) of the dedicated .recess 294 so that continued abduction of the femoral head is retarded. Conversely, as the femoral head 290 is rotated in a direction of adduction, the projection 292 moves closer to its adduction range of motion boundary so that continued rotation of the femoral head in the direction of adduction eventually causes the projection to contact another edge of the dedicated recess 294 so that continued adduction of the femoral head is retarded. Exemplary purposes of the projection 292 and recess 294 include, without limitation: (1) to replicate the constraint provided to the hip joint by the femoral head ligament; and, (2) to constrain the femoral head to attempt to maintain the femoral head 290 in the anatomical hip center.

In exemplary form, the femoral head 290 comprises a ball having a bearing surface with an arcuate curvature that mimics a sphere. This bearing surface is intended to contact a corresponding bearing surface on the interior of the acetabular cup liner 296, which is itself arcuate and mimics the curvature of a sphere (in this case slightly larger than the femoral head sphere), but in a convex orientation to allow the femoral ball to rotate and pivot within the femoral cup liner. Consequently, the bearing surface of the femoral ball 290 rides against the bearing surface of the acetabular cup liner 296. But, unlike preexisting acetabular cup liners and femoral balls, both components 290, 296 include a corresponding projection 292 and recess 294 to retard the range of motion of the femur with respect to the pelvis in the abduction and adduction directions.

In exemplary form, the femoral head 290 includes the projection 292. It should be noted that the projection 292 may be of any size and shape such as, without limitation, spherical, square, rectangular, triangular, and irregular. In this exemplary embodiment, the projection 292 is provided on the femoral head 290 proximate the radial direction where the femoral head ligament was previously located on the femoral head. However, this is only one exemplary location for the projection 292. Other locations will likewise be acceptable that allow for positioning of the projection in locations that are or are not weight bearing. FIGS. 41 shows two exemplary positions the projection 292 may occupy on the femoral ball 290 with respect to the weight bearing portion of the femoral ball.

In counterpart fashion, the acetabular cup liner 296 includes the recess 294 that at least partially receives the projection 292 of the femoral ball 290. It should be noted that the recess 294 may be of any concave shape such as, without limitation, spherical, square, rectangular, triangular, and irregular. In this exemplary embodiment, the recess 294 is provided on the acetabular cup liner 296 proximate the radial direction where the femoral head ligament was previously coupled to the pelvis. Depending upon exemplary considerations such as patient age and weight, the recess 294 may be approximately the same size as the projection 292 or may be greater than an order of magnitude than the projection. As will be understood by those skilled in the art, the greater the ratio between the size of the recess 294 and the size of the projection 292 (presuming a semispherical projection and recess) the greater the range of motion will be available to the patient in at least one of the abduction and adduction directions (i.e., both rotation and translation of the femoral ball with respect to the acetabular cup insert may occur). In other words, translation is limited to the amount of clearance between the projection 292 and the recess 294. Conversely, when the projection 292 and the recess 294 are approximately the same size, significant motion will be retarded in both the abduction and adduction directions (i.e., only significant rotation, but not translation, of the femoral ball with respect to the acetabular cup insert may occur).

Referencing FIGS. 43 and 44, in a further exemplary embodiment 300, a projection 302 is located on an acetabular cup liner 304 and a recess 306 is located on a femoral head 308. Just as with the prior exemplary embodiment 288, this exemplary embodiment 300 uses the interaction of a projection 302 and a recess 306 to retard the motion of the femoral ball 308 in at least one of the adduction and abduction directions. This projection on the acetabular cup could also be located on the acetabular shell and protruded through the acetabular liner. The projection could be of the same material as the acetabular cup and/or liner or could be of a different material.

The femoral ball 308 includes a recess 306 the recess that at least partially receives the projection 302 of the acetabular cup liner 304. It should be noted that the recess 306 may be of any concave shape such as, without limitation, spherical, square, rectangular, triangular, and irregular. In this exemplary embodiment, the recess 306 is provided on the femoral ball 308 proximate the radial direction where the femoral head ligament was previously located on the femoral head. Depending upon exemplary considerations such as patient age and weight, the recess 306 may be approximately the same size as the projection or may be greater than an order of magnitude than the projection. As will be understood by those skilled in the art, the greater the ratio between the size of the recess 306 and the size of the projection 302 (presuming a semispherical projection and recess) the greater the range of motion will be available to the patient in at least one of the abduction and adduction directions (i.e., both rotation and translation of the femoral ball 308 with respect to the acetabular cup insert may occur). In other words, translation is limited to the amount of clearance between the projection 302 and the recess 306. Conversely, when the projection 302 and the recess 306 are approximately the same size, significant motion will be retarded in both the abduction and adduction directions (i.e., only significant rotation, but not translation, of the femoral ball 308 with respect to the acetabular cup liner 304 may occur).

Conversely, the acetabular cup liner 304 includes the projection 302. It should be noted that the projection 302 may be of any shape such as, without limitation, spherical, square, rectangular, triangular, and irregular. In this exemplary embodiment, the projection 302 is provided on the femoral head proximate the radial direction where the femoral head ligament was previously coupled to the pelvis. However, this is only one exemplary location for the projection 302. Other locations will likewise be acceptable that allow for positioning of the projection 302 in locations that are or are not weight bearing. FIG. 43 shows two exemplary positions the projection 302 may occupy on the acetabular cup liner 304 with respect to the weight bearing portion of the femoral ball 308.

Following from the above description and invention summaries, it should be apparent to those of ordinary skill in the art that, while the apparatuses herein described constitute exemplary embodiments of the present invention, the invention is not limited to the foregoing and changes may be made to such embodiments without departing from the scope of the invention as defined by the claims. Additionally, it is to be understood that the invention is defined by the claims and it is not intended that any limitations or elements describing the exemplary embodiments set forth herein are to be incorporated into the interpretation of any claim element unless such limitation or element is explicitly stated. Likewise, it is to be understood that it is not necessary to meet any or all of the identified advantages or objects of the invention disclosed herein in order to fall within the scope of any claims, since the invention is defined by the claims and since inherent and/or unforeseen advantages of the present invention may exist even though they may not have been explicitly discussed herein.

## Claims

1. A prosthetic femoral component (100) comprising:
a femoral shaft (118) adapted to be received within an an intercondylar channel of a patient's femur,
a femoral neck (120, 184) operatively coupled to the femoral shaft and including a trunnion (122), and
a femoral ball (186),
**characterised in that** a neck axis extending longitudinally through the femoral neck is angled between zero and 10 degrees with respect to the femoral shaft.

2. The prosthetic femoral component (100) of claim 1, in which the neck axis extending longitudinally through the femoral neck (120, 184) is angled between ten and five degrees with respect to the femoral shaft axis extending longitudinally through the femoral shaft (118).

3. The prosthetic femoral component (100) of claim 1, further comprising a retention bracket adapted to couple to the femoral shaft (118) using a fastener, and in which the retention bracket is optionally adapted to be positioned on an external surface of a femur.

## Patentansprüche

1. Prothetische Femurkomponente (100), umfassend:
einen Femurschaft (118), der angepasst ist, in einem interkondylären Kanal eines Oberschenkels eines Patienten aufgenommen zu werden,
einen Femurhals (120, 184), der operativ mit dem Femurschaft gekoppelt ist und einen Zapfen (122) enthält, und
eine Femurkugel (186),
**dadurch gekennzeichnet, dass** eine Halsachse, die sich longitudinal durch den Femurhals erstreckt, zwischen null und 10 Grad in Bezug auf den Femurschaft abgewinkelt ist.

2. Prothetische Femurkomponente (10) nach Anspruch 1, wobei die Halsachse, die sich longitudinal durch den Femurhals (120, 184) erstreckt, zwischen zehn und fünf Grad in Bezug auf die Femurschaftachse abgewinkelt ist, die sich longitudinal durch den Femurschaft (118) erstreckt.

3. Prothetische Femurkomponente (100) nach Anspruch 1, ferner umfassend eine Rückhaltehalterung, die angepasst ist, unter Verwendung eines Befestigungsmittels an den Femurschaft (118) zu koppeln, und wobei die Rückhaltehalterung optional angepasst ist, an einer externen Fläche bzw. Oberfläche eines Femur positioniert zu werden.

## Revendications

1. Composant prothétique fémoral (100) comprenant :
une tige fémorale (118) adaptée pour être reçue dans un canal intercondylaire du fémur d'un patient,
un col fémoral (120, 184) couplé fonctionnellement à la tige fémorale et incluant un tourillon (122), et
une boule fémorale (186),
**caractérisé en ce qu'**un axe de col s'étendant longitudinalement au travers du col fémoral présente un angle entre zéro et 10 degrés par rapport à la tige fémorale.

2. Composant prothétique fémoral (100) selon la revendication 1, dans lequel l'axe du col s'étendant longitudinalement au travers du col fémoral (120, 184) présente un angle entre dix et cinq degrés par rapport à l'axe de tige fémorale s'étendant longitudinalement au travers de la tige fémorale (118).

3. Composant prothétique fémoral (100) selon la revendication 1, comprenant en outre un support de retenue adapté pour coupler la tige fémorale (118) à l'aide d'un élément de fixation, et dans lequel le support de retenue est facultativement adapté pour être positionné sur une surface externe d'un fémur.
